# EUROPEAN PATENT APPLICATION

(11) **EP 2 289 444 A1**
(43) Date of publication of application: **02.03.2011**
(21) Application number: 10173972.0
(22) Date of filing: 25.08.2010
(51) Int. Cl.: A61B 18/12, A61B 18/18, A61B 6/03, G01R 33/48, A61B 19/00

(54) **System for performing an electrosurgical procedure using an imaging compatible electrosurgical system**

(30) Priority: 25.08.2009 US 547120
(71) Applicant: Tyco Healthcare Group, LP, Boulder, CO 80301 (US)
(72) Inventor: Prakash, Mani N., Boulder, CO 80301 (US); Behnke, Robert J., Erie, CO 80516 (US)
(74) Representative: HOFFMANN EITLE

(57) **Abstract**

A method for performing an electrosurgical procedure includes the steps of supplying energy from an energy source to tissue and continuously receiving, as input, an imaging signal generated by an imaging device adapted to image tissue. The method also includes modifying the supply of energy from the energy source to tissue based on the imaging signal.

## Description

### BACKGROUND

### Technical Field

The present disclosure relates to energy-based apparatuses, systems and methods. More particularly, the present disclosure is directed to a system and method for performing an electrosurgical procedure using an imaging compatible electrosurgical system.

### Background of Related Art

Energy-based tissue treatment is well known in the art. Various types of energy (e.g., electrical, ultrasonic, microwave, cryo, heat, laser, etc.) are applied to tissue to achieve a desired result. Electrosurgery involves application of high radio frequency electrical current to a surgical site to cut, ablate, coagulate or seal tissue. In monopolar electrosurgery, a source or active electrode delivers radio frequency energy from the electrosurgical generator to the tissue and a return electrode carries the current back to the generator. In monopolar electrosurgery, the source electrode is typically part of the surgical instrument held by the surgeon and applied to the tissue to be treated. A patient return electrode is placed remotely from the active electrode to carry the current back to the generator.

In the case of tissue ablation, high radio frequency electrical current is applied to a targeted tissue site to create an ablation volume. The resulting ablation volume may then be observed and various ablation metrics may be measured and recorded. Typically, ablation metrics are obtained as scanned data obtained through use of imaging devices such as CT, MRI, PET, or other tomographic or X-ray devices. However, images obtained using such scanning techniques during an electrosurgical procedure, such as tissue ablation, are often distorted due to interference from the generator, electrosurgical instruments, and cables or wires connecting the electrosurgical instruments to the generator.

### SUMMARY

According to an embodiment of the present disclosure, a method for performing an electrosurgical procedure includes the steps of supplying energy from an energy source to tissue and continuously receiving, as input, an imaging signal generated by an imaging device adapted to image tissue. The method also includes modifying the supply of energy from the energy source to tissue based on the imaging signal.

According to another embodiment of the present disclosure, a method for performing an electrosurgical procedure includes the step of supplying energy from a generator to one or more electrosurgical instruments adapted to apply energy to tissue. The method also includes the step of continuously receiving, as input, an imaging signal generated by an imaging device adapted to image tissue. The method also includes the step of modifying the supply of energy from the energy source to the electrosurgical instrument based on the imaging signal such that the supply of energy from the energy source to the electrosurgical instrument is either terminated or diverted to an electrical load.

According to another embodiment of the present disclosure, an electrosurgical system adapted for use with an imaging device includes an energy source adapted to supply energy to one or more electrosurgical instruments configured to apply energy to tissue and an imaging device operably coupled to the energy source and adapted to image tissue. The imaging device is configured to continuously generate an imaging signal. The supply of energy to the one or more electrosurgical instruments is either terminated or diverted to an electrical load based on the imaging signal.

### BRIEF DESCRIPTION OF THE DRAWINGS

Various embodiments of the present disclosure are described herein with reference to the drawings wherein:

Figs. 1A and 1B are schematic block diagrams of an electrosurgical system according to an embodiment of the present disclosure;

Fig. 1C is a schematic block diagram of an electrosurgical system according to another embodiment of the present disclosure;

Fig. 2 is a schematic block diagram of a generator according to one embodiment of the present disclosure;

Fig. 3A is a schematic block diagram of an electrosurgical system according to another embodiment of the present disclosure;

Fig. 3B is a timing diagram illustrating operation of the electrosurgical system of Fig. 3A;

Fig. 4A is a schematic block diagram of an electrosurgical system according to another embodiment of the present disclosure;

Fig. 4B is a timing diagram illustrating operation of the electrosurgical system of Fig. 4A; and

Fig. 5 is a flow chart illustrating a method for performing an electrosurgical procedure according to one embodiment of the present disclosure.

### DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail.

An electrosurgical generator according to the present disclosure can perform monopolar and bipolar electrosurgical procedures, including tissue ablation procedures. The generator may include a plurality of outputs for interfacing with various electrosurgical instruments (e.g., a microwave antenna, a monopolar active electrode, return electrode, bipolar electrosurgical forceps, footswitch, etc.). Further, the generator includes electronic circuitry configured to generate electrosurgical energy (e.g., RF, microwave, etc.) specifically suited for various electrosurgical modes (e.g., cut, coagulate, desiccate, fulgurate, etc.) and procedures (e.g., ablation, vessel sealing, etc.).

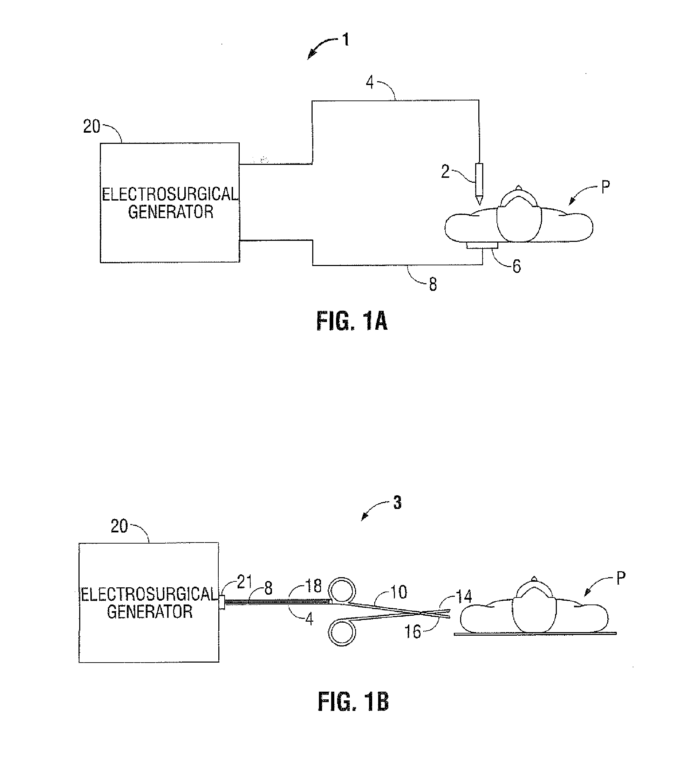

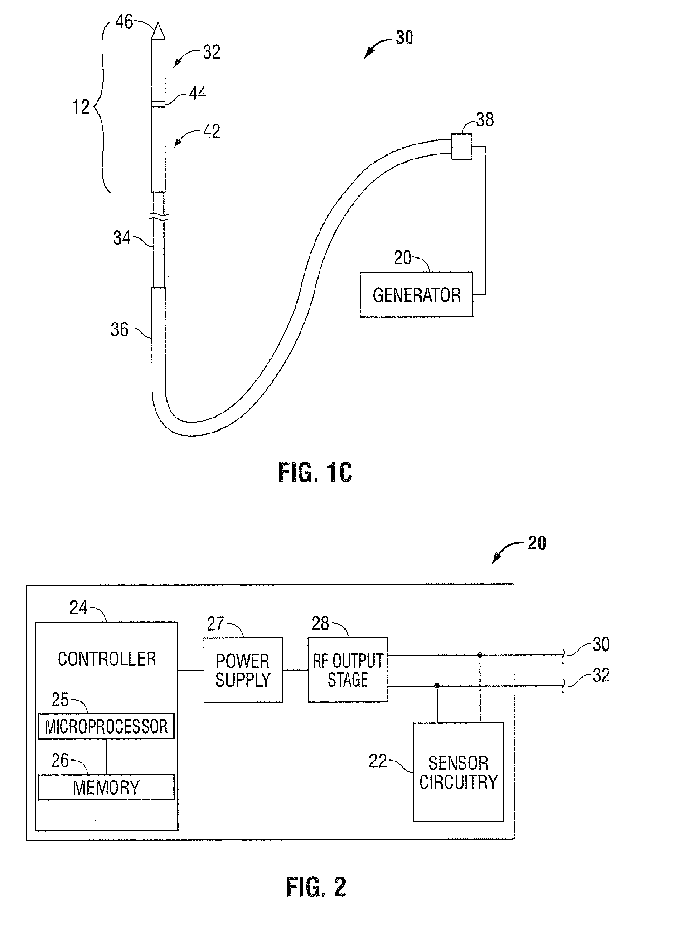

Fig. 1A is a schematic illustration of a monopolar electrosurgical system 1 according to one embodiment of the present disclosure. The system 1 includes an electrosurgical instrument 2 having one or more electrodes for treating tissue of a patient P. The instrument 2 is a monopolar type instrument including one or more active electrodes (e.g., electrosurgical cutting probe, ablation electrode(s), etc.). Electrosurgical energy is supplied to the instrument 2 by a generator 20 via a supply line 4, which is connected to an active terminal 30 (Fig. 2) of the generator 20, allowing the instrument 2 to coagulate, seal, ablate and/or otherwise treat tissue. The energy is returned to the generator 20 through a return electrode 6 via a return line 8 at a return terminal 32 (Fig. 2) of the generator 20.

Fig. 1B is a schematic illustration of a bipolar electrosurgical system 3 according to the present disclosure. The system 3 includes a bipolar electrosurgical forceps 10 having one or more electrodes for treating tissue of a patient P. The electrosurgical forceps 10 include opposing jaw members having an active electrode 14 and a return electrode 16, respectively, disposed therein. The active electrode 14 and the return electrode 16 are connected to the generator 20 through cable 18, which includes the supply and return lines 4, 8 coupled to the active and return terminals 30, 32, respectively (Fig. 2). The electrosurgical forceps 10 are coupled to the generator 20 at a connector 21 having connections to the active and return terminals 30 and 32 (e.g., pins) via a plug disposed at the end of the cable 18, wherein the plug includes contacts from the supply and return lines 4, 8.

Fig. 1C shows a diagram of an ablation antenna assembly 30 according to the present disclosure. In embodiments, antenna assembly 30 is a microwave antenna assembly adapted to deliver microwave energy from generator 20 to tissue. The antenna assembly 30 generally includes a radiating portion 12 that may be coupled by a feedline 34 (or shaft) via a conduit 36 to a connector 38, which may further connect the assembly 30 to the generator 20. Assembly 30 includes an ablation probe assembly (e.g., dipole antenna, helical antenna, etc.). A distal portion 32 of radiating portion 12 includes a tip 46 configured to allow for insertion into tissue with minimal resistance. A junction member 44 is located between a proximal portion 42 and distal portion 32 such that a compressive force may be applied by distal and proximal portions 44, 42 upon junction member 44.

Fig. 2 shows a schematic block diagram of the generator 20 having a controller 24, a high voltage DC power supply 27 ("HVPS") and an energy output stage 28 configured to output electrosurgical energy (e.g., microwave, RF, etc) from generator 20. The HVPS 27 is connected to a conventional AC source (e.g., electrical wall outlet) and provides high voltage DC power to the energy output stage 28, which then converts high voltage DC power into electrosurgical energy and delivers the electrosurgical energy to the active terminal 30. In some embodiments (Figs. 1A and 1B), the electrosurgical energy is returned to the energy output stage 28 via the return terminal 32.

The generator 20 may include a plurality of connectors to accommodate various types of electrosurgical instruments (e.g., instrument 2, electrosurgical forceps 10, antenna assembly 30, etc.). Further, the generator 20 may operate in monopolar or bipolar modes by including a switching mechanism (e.g., relays) to switch the supply of energy between the connectors, such that, for instance, when the instrument 2 is connected to the generator 20, only the monopolar plug receives electrosurgical energy.

The controller 24 includes a microprocessor 25 operably connected to a memory 26, which may be volatile type memory (e.g., RAM) and/or non-volatile type memory (e.g., flash media, disk media, etc.). The microprocessor 25 includes an output port that is operably connected to the HVPS 27 and/or the energy output stage 28 allowing the microprocessor 25 to control the output of the generator 20 according to either open and/or closed control loop schemes. Those skilled in the art will appreciate that the microprocessor 25 may be substituted by any logic processor or analog circuitry (e.g., control circuit) adapted to perform the calculations discussed herein.

Generally, the present disclosure relates to the use of a generator (e.g., generator 20) in an imaging setting or a so-called "MRT suite" setting. Specifically, electrosurgical energy (e.g., microwave, RF, etc.) generated by an electrosurgical generator is attracted to high-strength magnets employed by imaging devices or scanners (e.g., CT scanners, MRI scanners, etc.). This attraction causes distortions to image data generated by such imaging devices when energy is being generated in close proximity to the imaging device during an imaging procedure. This problem may be addressed by placing the generator outside the suite and running cables through the wall into the magnet area.

In one embodiment, image distortion is addressed using filters (e.g., notch filters) to minimize the interference between the generator and the imaging device. In this scenario, suitable filters may be incorporated within the generator and/or the imaging device.

In other embodiments, interference between the generator and the imaging device is minimized by modifying or affecting generator output such that operation of the generator is compatible with operation of the imaging device in the same procedure area and/or during the same procedure.

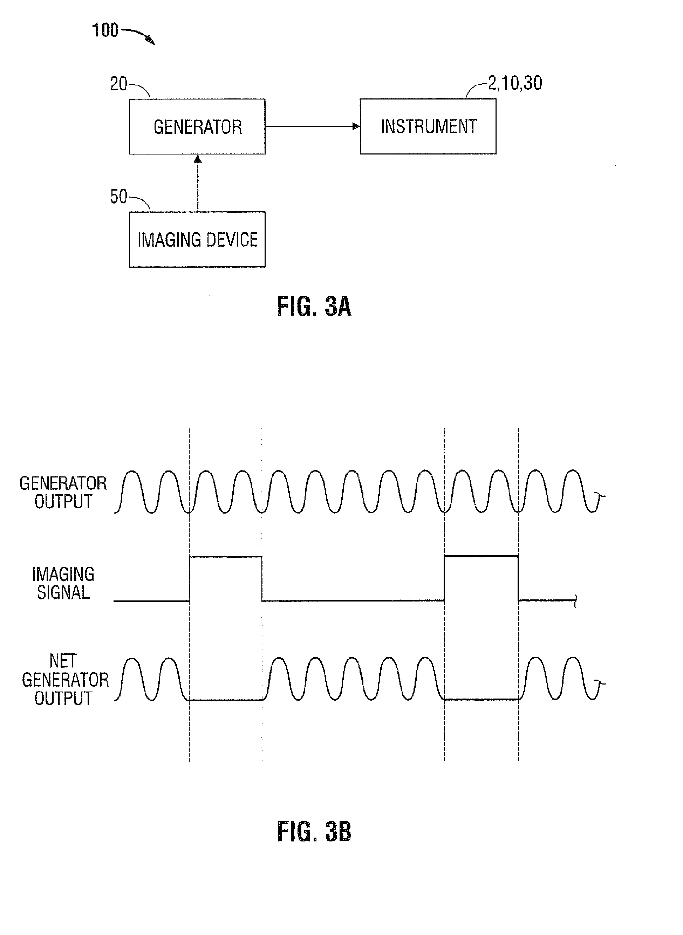

Fig. 3A illustrates an imaging compatible electrosurgical system 100 according to an embodiment of the present disclosure. Generally, system 100 includes generator 20 operably coupled to an imaging device 50 and an electrosurgical instrument, referenced as 2,10,30 to illustrate that instrument may be any one of instruments 2, 10, or 30 of Figs. 1A, 1B, or 1C, respectively. Imaging device 50 is adapted to image tissue and may be, for example without limitation, an imaging probe, an MRI device, a so-called "MRI suite", a CT device, a PET device, X-ray device, or any combination thereof. Imaging device 50 may include a processor operably coupled with a memory (not shown) storing any suitable imaging software and/or image processing software executable as programmable instructions by the processor to cause imaging device 50 to image tissue and/or generate tissue image data. In operation of system 100, generator 20 continuously receives an imaging signal generated by the imaging device 50 that is, in turn, processed by the controller 24. Based on the processed imaging signal, the controller 24 controls generator output. More specifically, the imaging signal generated by the imaging device 50 is a digital timing sequence configured to continuously indicate (e.g., via binary logic) in real-time whether or not an imaging sequence is currently being performed by the imaging device 50. Those skilled in the art will appreciate that imaging device 50 includes suitable circuitry (e.g., processor, memory, a/d converter, etc.) configured to generate the imaging signal as output and, further, that controller 24 and/or microprocessor 25 includes suitable circuitry configured to receive and process the imaging signal as input.

The imaging signal is processed by the controller 24 to cause the energy output stage 28 to terminate energy output from generator 20 during an imaging procedure and to allow energy output from the generator 20 while no imaging procedure is being performed by the imaging device 50. By way of example, Fig. 3B shows a timing diagram illustrating net generator output during continuous processing of the imaging signal by the generator 20 during operation thereof. As shown in the illustrated embodiment, while an imaging procedure is in progress, the imaging signal generated by the imaging device 50 is logic "high", indicating an imaging procedure is currently being performed by the imaging device 50. This, in turn, causes net generator output to the instrument 2, 10, 30 to be terminated and/or suspended by controller 24, as indicated by a logic "low" in the illustrated timing diagram. Likewise, while an imaging procedure is not currently in progress, the imaging signal generated by the imaging device 50 is logic "low', indicating that an imaging procedure is not currently in progress. The logic low is processed by the controller 24, which in turn, causes energy output phase 28 to output energy to the instrument 2, 10, 30 as indicated by a logic "high" in the illustrated timing diagram. In this manner, imaging procedures (e.g., MRT) and electrosurgical procedures (e.g., ablation) may be performed in close proximity and essentially during the same procedure or operation without adverse effects (e.g., image distortion) to the imaging process caused by interference from the generator 20, instrument 2, 10, 30 and/or cables therebetween.

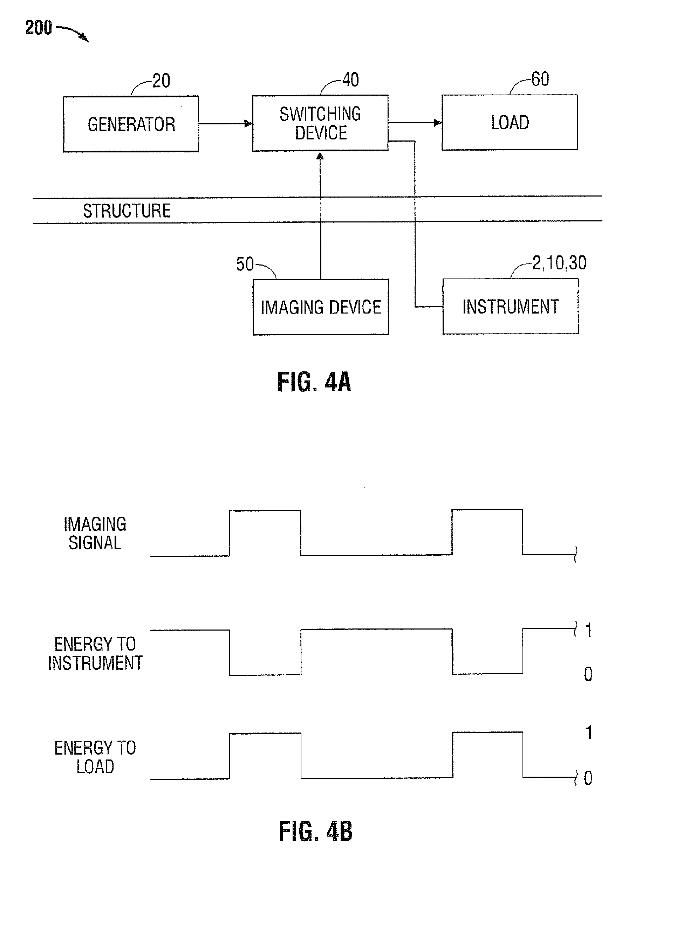

Fig. 4A illustrates an imaging compatible electrosurgical system 200 according to another embodiment of the present disclosure. In this embodiment, a switching device 40 is incorporated between the generator 20 and the instrument 2, 10, 30. The switching device 40 is configured to continuously receive the imaging signal from the imaging device 50 in substantially the same manner as described above with respect to the embodiment of Figs. 3A and 3B. The switching device 40 may be, for example, an electromechanical switch activated by the imaging signal generated by the imaging device 50. With this purpose in mind, switching device 40 includes any one or more suitable switching components and includes circuitry configured to receive and process the imaging signal from the imaging device 50 as input. Generally, the switching device 40 receives and processes the imaging signal generated by the imaging device 50 and switches generator output between instrument 2, 10, 30 and an electrical load 60 based on the processed imaging signal to substantially eliminate interference with the imaging device 50 caused by generator 20, instrument 2, 10, 30 and cable or wire connections therebetween during an imaging procedure. More specifically, while an imaging procedure is in progress, switching device 40 diverts generator output from instrument 2, 10, 30 to electrical load 60. Likewise, while an imaging procedure is not in progress, switching device 40 diverts generator output from electrical load 60 to instrument 2, 10, 30. By way of example, Fig. 4B shows a timing diagram illustrating the switching of generator output between instrument 2, 10, 30 and electrical load 60 during continuous processing of the imaging signal by the switching device 40 during an electrosurgical procedure. As shown in the illustrated embodiment, while an imaging procedure is currently being performed by the imaging device 50, the imaging signal generated by the imaging device 50 is logic "high", indicating that an imaging procedure is currently being performed. As illustrated by the timing diagram, this, in turn, causes switching device 40 to switch the path of generator output away from the instrument 2, 10, 30, as indicated by a logic "low", to electrical load 60, as indicated by a logic "high". While an imaging procedure is not currently being performed by the imaging device 50, the imaging signal generated by the imaging device 50 is logic "low", indicating that an imaging procedure is not currently in being performed by the imaging device 50. As illustrated by the timing diagram, this, in turn, causes switching device 40 to switch the path of generator output away from electrical load 60, as indicated by a logic "low", to instrument 2, 10, 30, as indicated by a logic "high". In this and other embodiments, generator 20, switching device 40, electrical load 60, and any cable or wire connections therebetween, may be located in a room separate from the room where imaging device 50 and instrument 2, 10, 30 are located (e.g., operating room). In this scenario, any cable or wire connections between switching device 40 and imaging device 50 and/or instrument 2, 10, 30 may be passed through a structure (e.g., wall, door, floor, ceiling, etc.) from one room to another. In this manner, when switching device 40 (located outside the procedure room) diverts energy from instrument 2, 10, 30 to electrical load 60 during an imaging procedure, no energy is being transferred between switching device 40 and instrument 2, 10, 30 (inside the procedure room), thereby substantially eliminating interference with imaging device 50 caused by generator 20, instrument 2, 10, and any cable or wire connections therebetween.

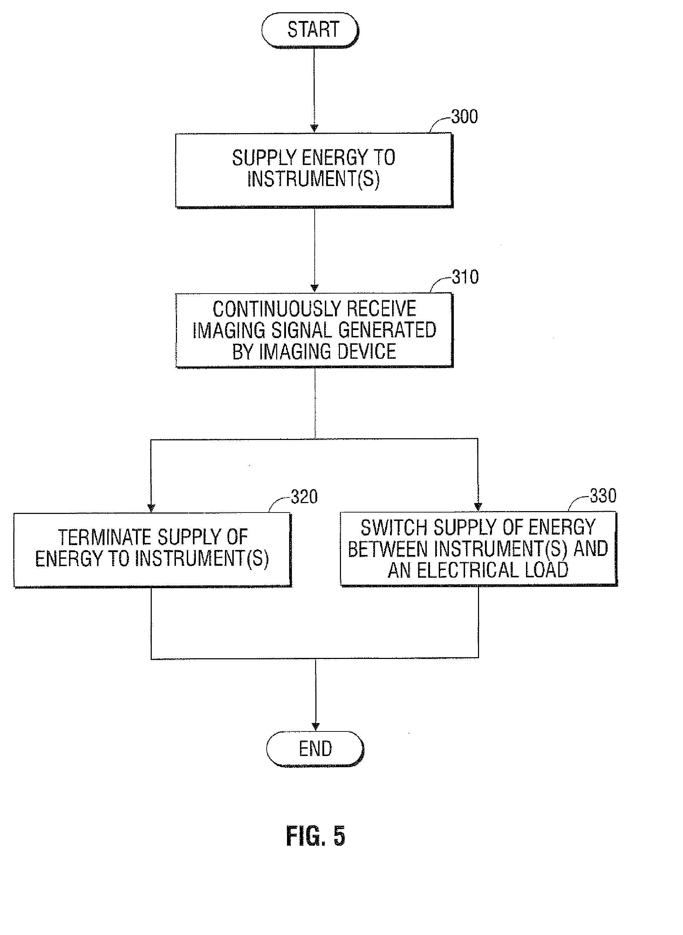

A method for performing an electrosurgical procedure using an imaging compatible energy source according to embodiments of the present disclosure will now be described with reference to Fig. 5 in conjunction with Figs. 1A-4B.

In step 300, electrosurgical energy is supplied from the generator 20 to the instrument (e.g., instrument 2, forceps 10, etc.). In embodiments, instrument 2, 10, 30 is used to apply energy from the generator 20 to tissue (e.g., to create an ablation lesion). In step 310, the imaging device 50 continuously generates an imaging signal, as illustrated in Figs. 3B and 4B.

In one embodiment, illustrated in Figs. 3A and 3B, the imaging signal is received and processed by the generator 20. In step 320, based on the received imaging signal, the controller 24 terminates generator output while the imaging device 50 is performing an imaging procedure and permits generator output when no imaging procedure is being performed by the imaging device 50.

In another embodiment, illustrated in Figs. 4A and 4B, the imaging signal is received and processed by the switching device 40. In step 330, based on the received imaging signal, the switching device 40 switches generator output between instrument 2, 10, 30 and electrical load 60. More specifically, the switching device 40 diverts generator output from the instrument 2, 10, 30 to the electrical load 60 while the imaging device 50 is performing an imaging procedure. Likewise, the switching device 50 diverts generator output from the electrical load 60 to the instrument 2, 10, 30 when no imaging procedure is being performed by the imaging device 50.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. For example, it should be understood that any of the above disclosed embodiments may be configured such that imaging device 50 generates a logic low to indicate an imaging procedure is currently being performed and, vice-versa, a logic high may indicate that no imaging procedure is currently being performed. Those skilled in the art will envision other modifications within the scope and spirit of the claims appended hereto.

## Claims

1. An electrosurgical system adapted for use with an imaging device, comprising:
an energy source adapted to supply energy to at least one electrosurgical instrument configured to apply energy to tissue; and
an imaging device operably coupled to the energy source and adapted to image tissue, the imaging device configured to continuously generate an imaging signal, wherein the supply of energy to the at least one electrosurgical instrument is one of terminated or diverted to an electrical load based on the imaging signal.

2. An electrosurgical system according to claim 1, wherein the imaging signal generated by the imaging device is received, as input, at the energy source.

3. An electrosurgical system according to claims 1, wherein the imaging signal generated by the imaging device is received, as input, at a switching device configured to switch energy supplied by the energy source between the electrosurgical instrument and the electrical load based on the imaging signal.

4. System according to claim 1, 2 or 3 including means for terminating the energy supplied from the energy source to tissue based on the imaging signal.

5. System according to any one of the preceding claims and including means for switching the energy supplied from the energy source to tissue between an electrosurgical instrument adapted to apply energy to tissue and an electrical load based on the imaging signal.

6. System according to any one of the preceding claims, wherein the imaging signal is a digital signal indicative of an imaging procedure status.

7. System according to any one of the preceding claims and including means for separating the imaging device from at least one of the energy source and an electrosurgical instrument adapted to supply energy to tissue such that at least one structure is disposed therebetween.

8. System according to any one of the preceding claims, wherein the electrosurgical instrument includes at least one electrode configured to conduct electrosurgical energy therethrough.

9. System according to any one of the preceding claims, wherein the electrosurgical instrument includes a radiating portion configured to conduct electrosurgical energy therethrough.

10. System according to any one of the preceding claims, wherein the electrosurgical instrument is one of a microwave antenna, a bipolar forceps, and a monopolar instrument including at least one active electrode.

11. System as in any one of the preceding claims, wherein the energy of the supplying step is RF energy.

12. System as in any one of claims 1 to 10, wherein the energy of the supplying step is microwave energy.

13. System according to any one of the preceding claims, wherein the imaging device is selected from ultrasound, CT, MRI, and PET imaging modalities.

14. Apparatus for performing an electrosurgical procedure, comprising:
means for supplying energy from a generator to at least one electrosurgical instrument adapted to apply energy to tissue;
means for continuously receiving, as input, an imaging signal generated by an imaging device adapted to image tissue; and
means for modifying the supply of energy from the energy source to the electrosurgical instrument based on the imaging signal, wherein the supply of energy from the energy source to the electrosurgical instrument is one of terminated or diverted to an electrical load.

15. System or apparatus as claimed in any one of the preceding claims wherein either the imaging signal of the continuously receiving step is received, as input, at the energy source, or at a switching device configured to switch energy supplied by the energy source between the electrosurgical instrument and the electrical load based on the imaging signal.
